(19) Europäisches Patentamt
European Patent Office
Office européen des brevets

(11) Veröffentlichungsnummer: **0 251 041**
**B1**

# (12) EUROPÄISCHE PATENTSCHRIFT

(45) Veröffentlichungstag der Patentschrift: **08.11.89**

(51) Int. Cl.⁴: **C07C 63/70**, C07C 51/60, C07C 51/04, C07C 51/093, C07C 25/13

(21) Anmeldenummer: **87108741.7**

(22) Anmeldetag: **19.06.87**

## (54) Verfahren zur Herstellung von 2,3,5,6-Tetrafluorbenzoesäure und die Verbindungen 2,3,5,6-Tetrachlor-4-trifluormethyl-benzoylchlorid und 2,3,5,6-Tetrafluor-4-trifluormethyl-benzoylfluorid.

(30) Priorität: **28.06.86 DE 3621707**

(43) Veröffentlichungstag der Anmeldung: **07.01.88 Patentblatt 88/1**

(45) Bekanntmachung des Hinweises auf die Patenterteilung: **08.11.89 Patentblatt 89/45**

(84) Benannte Vertragsstaaten: **BE CH DE FR GB IT LI NL**

(56) Entgegenhaltungen: **US-A- 3 459 794**

(73) Patentinhaber: **BAYER AG, D-5090 Leverkusen 1 Bayerwerk(DE)**

(72) Erfinder: **Kysela, Ernst, Dr., Virchowstrasse 14, D-5060 Bergisch Gladbach 1(DE)**
Erfinder: **Braden, Rudolf, Dr., Nothauser Feld 1, D-5068 Odenthal(DE)**

EP 0 251 041 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von 2,3,5,6-Tetrafluorbenzoesäure aus 4-Trifluormethyl-benzoylfluorid und die neuen Verbindungen 2,3,5,6-Tetrachlor-4-trifluormethyl-benzoylchlorid und 2,3,5,6-Tetrafluor-4-trifluormethyl-benzoylfluorid.

2,3,5,6-Tetrafluorbenzoesäure ist eine bekannte Verbindung, die beispielsweise zur Herstellung hochwirksamer Insektizide verwendet werden kann (siehe DE-OS 2 658 074). Die bekannten Verfahren zur Herstellung dieser Verbindung sind, insbesondere für eine Durchführung in technischem Maßstab, mit schwerwiegenden Nachteilen behaftet. Sie benötigen beispielsweise schwer zugängliche Ausgangsstoffe, schwierig zu handhabende Reaktionen und Chemikalien und/oder sind wenig selektiv (siehe z.B. R.J. Harper et al., J.O.C. 29, 2385-9 (1964) - Hauptnachteil: Grignard-Reaktion; V.I. Vysocin et al., Zh. Obsh. Chim. 39, 1607-15 (1969) - Hauptnachteil: Verwendung von Lithium-Aluminium- Hydrid, G.G. Yakobson et al., Zh. Org. Khim. 10, 799-804 (1974) -Hauptnachteil: Verwendung von Antimon (V)-fluorid; EP-OS 60617 - Hauptnachteil: Verwendung von Butyllithium und D.J. Alsop et al., J. Chem. Soc. 1962, 1801-5 - Hauptnachteil: geringe Ausbeute).

Es wurde nun ein Verfahren zur Herstellung von 2,3,5,6-Tetrafluorbenzoesäure gefunden, daß dadurch gekennzeichnet ist, daß man

a) 4-Trifluormethyl-benzoylfluorid in Gegenwart von Chlorsulfonsäure und gegebenenfalls in Gegenwart eines Lösungsmittels bei Temperaturen von weniger als 60°C zu 2,3,5,6-Tetrachlor-4-trifluormethyl-benzoylchlorid chloriert.

b) 2,3,5,6-Tetrachlor-4-trifluormethyl-benzoylchlorid mit Kaliumfluorid in Gegenwart eines aprotischen, dipolaren, inerten Lösungsmittels zu 2,3,5,6-Tetrafluor-4-trifluormethyl-benzoylfluorid fluoriert,

c) 2,3,5,6-Tetrafluor-4-trifluormethyl-benzoylfluorid zu 2,3,5,6-Tetrafluor-4-trifluormethyl-benzoesäure hydrolysiert,

d) 2,3,5,6-Tetrafluor-4-trifluormethyl-benzoesäure bei erhöhter Temperatur zu 2,3,5,6-Tetrafluorbenzotrifluorid decarboxyliert und

e) 2,3,5,6-Tetrafluor-benzotrifluorid mit Oleum zu 2,3,5,6-Tetrafluor-benzoesäure verseift.

Die Produkte der Stufen b) und c) werden vorzugsweise nicht isoliert. Man verfährt vorzugsweise so, daß man Stufen c) und d) unmittelbar nacheinander und im selben Gefäß wie die Stufe b) ablaufen läßt ohen Isolierung des Produktes der Stufe b) und während oder nach der Hydrolyse Temperaturen einstellt, bei denen die Decarboxylierung stattfindet. Auf diese Weise kann man das erfindungsgemäße Verfahren als 3-Stufen-Reaktion ablaufen lassen.

Diese bevorzugte Ausführungsform des erfindungsgemäßen Verfahrens läßt sich beispielhaft durch folgendes Formelschema illustrieren:

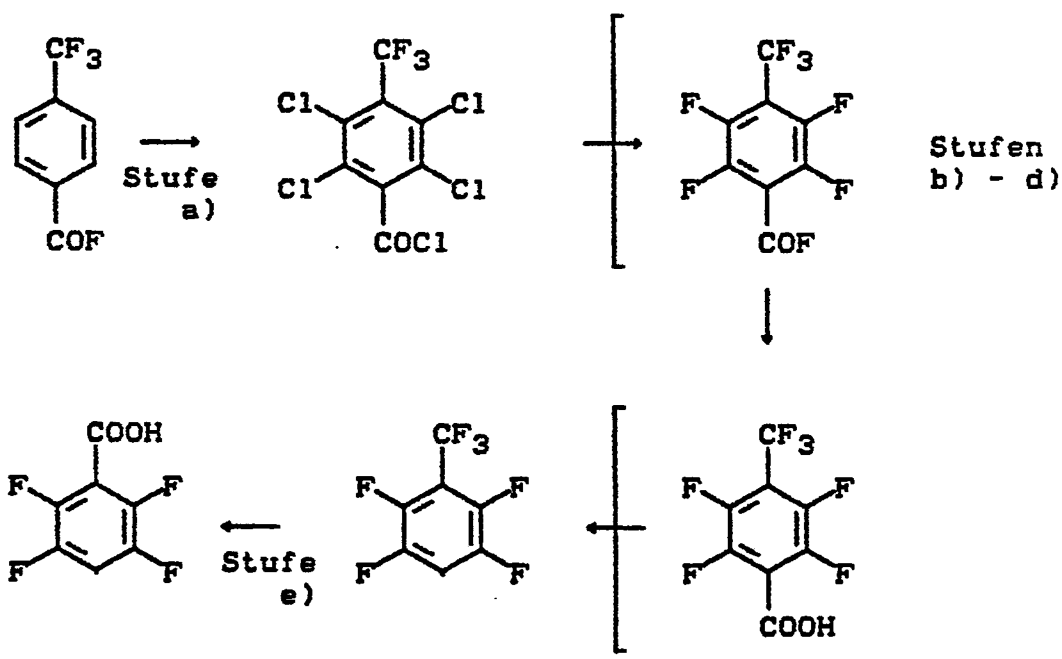

Das für die Stufe a) benötigte Ausgangsprodukte 4-Trifluormethyl-benzoylfluorid ist ein Handelsprodukt.

Chlorsulfonsäure kann beispielsweise in Mengen von 2 - 10 Mol pro Mol 4-Trifluormethyl-4-benzoylfluorid eingesetzt werden. Vorzugsweise beträgt diese Menge 4 - 7,5 Mol. Die Chlorierung in Stufe a) kann im allgemeinen mit elementarem Chlor durchgeführt werden.

Man kann beispielsweise so lange Chlor in das Reaktionsgemisch einleiten, wie es dort aufgenommen wird. Es kann vorteilhaft sein, dem Reaktionsgemisch einge geringe Menge Jod zuzusetzen, beispielsweise bezogen auf eingesetztes Chlor 0,01 -2 Gew.-%. Es kann weiterhin vorteilhaft sein, die Chlorierung in Gegenwart eines Lösungsmittels durchzuführen. Geignete Lösungsmittel sind beispielsweise Tetrachlorkohlenstoff, Tetrachlorethan, Difluortetrachlorethan und Sulfurylchlorid. Bevorzugt sind 1,1,2,2-Tetrachlorethan und Sulfurylchlorid, von denen man beispielsweise 100 -500 Gew.-%, bezogen auf 4-Trifluormethylbenzoylfluorid, einsetzen kann. Bevorzugte Temperaturen bei der Chlorierung sind z.B. solche im Bereich von 40 bis 60°C, insbesondere solche im Bereich von 40 bis 55°C. Man kann grundsätzlich auch bei Temperaturen über 60°C arbeiten, jedoch nimmt dabei die Ausbeute an dem gewünschten Chlorierungsprodukt stark ab.

Die Aufarbeitung des nach der Chlorierungsreaktion der Stufe a) vorliegenden Reaktionsgemisches kann beispielsweise so erfolgen, daß man es, gegebenenfalls nach vorheriger Abkühlung, in ein Eis-Wasser-Gemisch einbringt und 2,3,5,6-Tetrachlor-4-trifluormethyl-benzoylchlorid mit Hilfe eines organischen Lösungsmittels abtrennt. Bei diesem organischen Lösungsmittel kann es sich um ein gegebenenfalls schon während der Reaktion vorgelegenes Lösungsmittel handeln, z.B. Tetrachlorkohlenstoff. Organisches Lösungsmittel kann aber auch erst oder zusätzlich bei der Aufarbeitung hinzugefügt werden. Durch Abtrennung der organischen Phase und deren Einengung bis zur Trockene läßt sich dann ein als Einsatzprodukt in die Stufe b) geeignetes 2,3,5,6-Tetrachlor-4-trifluormethyl-benzoylchlorid gewinnen. Falls der Chlorierungsreaktion Jod zugesetzt worden ist, entfernt man dieses zweckmäßigerweise vor der Abtrennung des 2,3,5,6-Tetrachlor-4-trifluormethyl-benzoylchlorids, z.B. durch Zugabe von Natriumhydrogensulfit.

Die Fluorierung in Stufe b) des erfindungsgemäßen Verfahrens kann beispielsweise mit 5 - 20 Mol Kaliumfluorid pro Mol 2,3,5,6-Tetrachlor-4-trifluormethyl-benzoylchlorid durchgeführt werden. Vorzugsweise beträgt diese Menge 6 - 10 Mol. Es kann auch geringe Mengen Wasser enthaltendes Kaliumfluorid eingesetzt werden. In diesem Falle ist es vorteilhaft, zunächst nur das Lösungsmittel und Kaliumfluorid zusammenzufügen und das Wasser dadurch zu entfernen, daß man eine kleine Menge Lösungsmittel zusammen mit dem Wasser destilliert. Das Lösungsmittel kann beispielsweise, bezogen auf 2,3,5,6-Tetrachlor-4-trifluormethyl-benzoylchlorid, in der 1 - 5-fachen Gewichtsmenge eingesetzt werden, vorzugsweise in der 1,5 -5-fachen Gewichtsmenge. Die Stufe b) des erfindungsgemäßen Verfahrens kann beispielsweise bei Temperaturen von 150 bis 200°C durchgeführt werden. Bevoruzgt sind Temperaturen von 160 bis 190°C. Geeignete Reaktionszeiten bei den bevorzugten Temperaturen sind beispielsweise solche von 12 bis 20 Stunden. Ein bevorzugtes Lösungsmittel für diese Stufe ist Tetramethylensulfon.

Vorzugsweise läßt man die Stufen c) und d) des erfindungsgemäßen Verfahrens unmittelbar aufeinander ablaufen und setzt dazu einfach dem gesamten nach der Stufe b) vorliegenden Reaktionsgemisch, vorzugsweise nach dessen Abkühlung auf eine Temperatur von 100°C oder weniger, Wasser zu, beispielsweise in der äquimolaren Menge oder einem molaren Überschuß, beispielsweise zum 15-fachen der äquimolaren Menge, jeweils bezogen auf das Edukt.

Die Decarboxylierung (Stufe d) des erfindungsgemäßen Verfahrens) setzt im allgemeinen bei Temperaturen um 80°C ein, d.h. gegebenenfalls schon während der Wasserzugabe. Vorzugsweise führt man die Decarboxylierung bei Temperaturen im Bereich von 100 bis 160°C (Sumpftemperatur) zu Ende.

Bereits während der Decarboxylierung kann gebildetes 2,3,5,6-Tetrafluor-benzotrifluorid abdestillieren. Zweckmäßigerweise fängt man dieses und alle bis zum Siedepunkt des Lösungsmittels (z.B. des Tetramethylensulfons) übergehenden, flüchtigen Komponenten gemeinsam auf. Da so erhaltenes rohes 2,3,5,6-Tetrafluor-benzotrifluorid im allgemeinen noch Wasser und Tetramethylensulfon enthält ist es im allgemeinen vorteilhaft, es mit wenig Wasser zu waschen und anschließend zu trocknen.

In die Stufe e) des erfindungsgemäßen Verfahrens kann 2,3,5,6-Tetrafluor-benzotrifluorid beispielsweise so eingesetzt werden, wie es nach der oben beschriebenen Aufarbeitung des Reaktionsgemisches aus Stufe d) anfällt. Das Oleum kann beispielsweise von 10 bis 50 Gew.-% $SO_3$ enthalten. Vorzugsweise enthält es 20 bis 30 Gew.-% $SO_3$. Das Oleum kann beispielsweise in einer Menge von einem Mol oder mehr $SO_3$, bezogen auf das Produkt der Stufe d), eingesetzt werden. Größere Überschüsse an $SO_3$, z.B. solche von der 4-fachen molaren Menge oder mehr, stören im allgemeinen nicht, sind jedoch unwirtschaftlich. Geeignete Temperaturen zur Durchführung der Stufe e) sind beispielsweise solche von 70 bis 120°C, geeignete Reaktionszeiten beispielsweise solche von 2 bis 5 Stunden.

Die Isolierung der so hergestellten 2,3,5,6-Tetrafluorbenzoesäure kann beispielsweise erfolgen, indem man das Reaktionsgemisch nach Abkühlung in ein Eis-Wasser-Gemisch einrührt, den sich bildenden Niederschlag abfiltiert, gegebenenfalls mit wenig kaltem Wasser wäscht und trocknet.

Auf diese Weise kann man unter Vermeidung der Nachteile der bekannten Verfahren 2,3,5,6-Tetrafluor-benzoesäure in Ausbeuten von beispielsweise 50 - 60 % d.Th. (bezogen auf eingesetztes 4-Trifluormethyl-benzoylfluorid) und in Reinheiten von über 95 %, häufig 98 %, herstellen. Es ist ausgesprochen überraschend, daß mit der vorliegenden Erfindung ein Verfahren zur Herstellung von 2,3,5,6-Tetrafluor-benzoesäure zur Verfügung gestellt wird, das nicht nur relativ gut zugängliche Ausgangsstoffe und relativ einfach zu handhabende Reaktionen und Chemikalien benötigt, sondern bei dem das gewünschte Produkt auch noch in relativ hohen Ausbeuten und in ausgezeichneten Reinheiten erhalten wird, denn beim erfindungsgemäßen Verfahren, das im einfachsten Fall 3 Reaktionsstufen umfaßt, war mit dem Ablauf mit Nebenreaktion in erheblichen Umfang zu rechnen. Es war beispielsweise zu erwarten, daß sich in Stufe a) die $CF_3$-Gruppe nicht so relativ inert verhält, sondern bei der Chlorierung und/oder Aufarbeitung mitreagiert und dadurch in erheblichen Umfang unerwünschte Nebenprodukte entstehen. Beispielsweise können durch Chlorierung aus $CF_3$-Gruppen $CF_2Cl$- und/oder $CFCl_2$-Gruppen enthaltende Nebenprodukte entstehen, durch die Hydrolyse von $CF_3$-Gruppen 2,3,5,6-Tetrachlor-terephthaloylchlorid und/oder durch nicht vollständige Chlorierung des aromatischen Kerns Tri-und/oder Dichlor-4-trifluormethyl-benzoylchlorid. Überaschend ist weiterhin, daß man nach Durchführung des erfindungsgemäßen Verfahrens auf einfache Weise eine sehr reine 2,3,5,6-Tetrafluorbenzoesäure isolieren kann, auch wenn die auftretenden Zwischenprodukte nicht besonders gereinigt werden und dort entstandene Nebenprodukte bis in die Stufe e) mitgeschleppt werden.

Die vorliegende Erfindung betrifft auch die bisher unbekannte Verbindung 2,3,5,6-Tetrachlor-4-trifluormethyl-benzoylchlorid. Diese kann erhalten werden, indem man das zuvor beschriebene erfindungsgemäße Verfahren nach der Stufe a) abbricht, das Reaktionsgemisch wie dort beschrieben aufarbeitet und gegebenenfalls weiter reinigt.

2,3,5,6-Tetrachlor-4-trifluormethyl-benzoylchlorid kann als Zwischenprodukt für die vorteilhafte Herstellung von 2,3,5,6-Tetrafluor-benzoesäure verwendet werden (siehe oben), das seinerseits ein Zwischenprodukt für hochwirksame Insektizide ist (siehe DE-OS 2 658 074).

Die vorliegende Erfindung betrifft weiterhin die bisher unbekannte Verbindung 2,3,5,6-Tetrafluor-4-trifluormethyl-benzoylfluorid. Diese kann erhalten werden, indem man das zuvor beschriebene erfindungsgemäße Verfahren nach der Stufe b) abbricht und das Reaktionsgemisch beispielsweise durch Destillation aufarbeitet.

2,3,5,6-Tetrafluor-4-trifluormethyl-benzoylfluorid kann als Zwischenprodukt für die vorteilhafte Herstellung von 2,3,5,6-Tetrafluorbenzoesäure verwendet werden (siehe oben), die ihrerseits ein Zwischenprodukt für hochwirksame Insektizide ist (siehe DE-OS 2 658 074).

## Beispiele

### Beispiel 1

a) In einer Rührapparatur aus Glas von 2 l Inhalt wurden 1 kg Tetrachlorkohlenstoff, 870 g Chlorsulfonsäure, 288 g 4-Trifluormethyl-benzoylfluorid und 6 g Jod vorgelegt und bei einer Temperatur von 55 - 60°C Chlor in diese Mischung eingeleitet. Nachdem 700 g Chlor eingeleitet waren, wurde das Gemisch auf Raumtemperatur abgekühlt, unter Kühlen in eine Mischung aus 2 kg Eiswasser und 800 g Tetrachlorkohlenstoff eingerührt und wässrige Natriumhydrogensulfitlösung zugegeben, bis das Jod entfernt war (ca. 20 g 40 %ige Lösung). Danach wurde die organische Phase abgetrennt, die wässrige Phase nochmals mit 200 g frischem Tetrachlorkohlenstoff gewaschen und aus den vereinigten Tetrachlorkohlenstoff-Extrakten der Tetrachlorkohlenstoff am Rotationsverdampfer entfernt. So wurden 505 g 83 % reines 2,3,5,6-Tetrachlor-4-Trifluormethyl-benzoylchlorid mit einem Schmelzpunkt von 72 - 76°C erhalten. Das entspricht einer Ausbeute an reinem Produkt von 80,5 % d.Th.

b) 1680 g Tetramethylensulfon und 600 g handelsübliches Kaliumfluorid wurden in eine Rührapparatur aus Glas von 2 l Inhalt gegeben, die mit einem Destillationsaufsatz versehen war. Durch Abdestillieren von 40 g Tetramethylensulfon wurde im Kaliumfluorid enthaltene Feuchtigkeit entfernt. Danach wurde bei 90°C Innentemperatur 505 g des gemäß a) erhaltenen Produktes zugegeben und 14 Stunden auf 180°C erhitzt. Danach wurde auf 100°C abgekühlt, und 44 g Wasser wurden zugetropft, wobei die Entwicklung von Kohlendioxid einsetzte. Nach Beendigung der Wasserzugabe wurde 15 Minuten nachgerührt, dann langsam bei nicht zu heftiger Kohlendioxidentwicklung auf 160°C erwärmt und das übergehende Destillat aufgefangen. Nach Beendigung der Kohlendioxidentwicklung wurde Vakuum angelegt und das bis 140°C bei 20 mbar übergehende Destillat aufgefangen. Die vereinigten Destillate wurden mit wenig Wasser gewaschen, anschließend getrocknet und nochmals destilliert. So wurden 216 g zu 88 % reines 2,3,5,6-Tetrafluor-benzotrifluorid mit einem Siedepunkt von 112°C erhalten. Das entspricht einer Ausbeute an reinem Produkt von 72 % d.Th.

c) 434 g 20 %iges Oleum und 216 g gemäß b) erhaltenes Produkt wurden in einem Glasgefäß vermischt und unter Rühren auf Rückflußtemperatur (103°C) erhitzt. Beim Nachlassen des Rückflußes wurde die Innentemperatur langsam auf 120°C gesteigert. Nach 3 Stunden wurde das Reaktionsgemisch auf 3°C abgekühlt und unter Kühlung in 200 g Eiswasser eingerührt, wobei die Temperatur unter 30 °C gehalten wurde. Die sich bildende Suspension wurde auf 3°C abgekühlt, der Niederschlag abfiltiert, und mit wenig Eiswasser gewaschen und getrocknet. So wurden 166 g 2,3,5,6-Tetrafluor-benzoesäure mit einem Schmelzpunkt von 148°C erhalten, was 97 % d.Th. entspricht. Die Reinheit der so isolierten 2,3,5,6-Tetrafluorbenzoesäure betrug über 99 %.

Beispiel 2

Es wurde verfahren wie Beispiel 1 a) jedoch wurde das so erhaltene 2,3,5,6-Tetrachlor-4-trifluormethyl-benzoylchlorid noch weiter gereinigt, indem es aus einem Gemisch aus Tetrachlorkohlenstoff und Petrolether umkristallisiert wurde.
So wurden 390 g eines 98 % reinen 2,3,5,6-Tetrachlor-4-trifluormethyl-benzoylchlorids mit einem Schmelzpunkt von 76°C erhalten.

Beispiel 3

Es wurde verfahren wie in Beispiel 1 b), jedoch wurde nach der Umsetzung mit Kaliumfluorid kein Wasser bei 100°C zugegeben, sondern das Reaktionsgemisch destilliert bis 140°C bei 20 mbar und anschließend redestilliert.
So wurden 133 g eines 92 % reinen 2,3,5,6-Tetrafluor-4-trifluormethyl-benzoylfluorids mit einem Siedepunkt von 54-56°C/20 mbar erhalten.

Beispiel 4

Es wurde verfahren wie in Beispiel 1a), jedoch wurden anstelle von Tetrachlorkohlenstoff 1,1 kg 1,1,2,2-Tetra chlorethan eingesetzt und das Chlor bei 45 bis 50°C eingeleitet. Es wurden 511 g 95 % reines 2,3,5,6-Tetrachlor-4-trifluormethylbenzoylchlorid mit einem Schmelzpunkt von 75-76°C erhalten. Das entspricht einer Ausbeute von 92,5 % d.Th.

Beispiel 5

Es wurde verfahren wie in Beispiel 4, jedoch wurde anstelle von Tetrachlorethan 900 g Sulfurylchlorid eingesetzt. Das Ergebnis entsprach Beispiel 4.

**Patentansprüche**

1) Verfahren zur Herstellung von 2,3,5,6-Tetraflurobenzoesäure, dadurch gekennzeichnet, daß man
a) 4-Trifluormethyl-benzoylfluorid in Gegenwart von Chlorsulfonsäure und gegebenenfalls in Gegenwart eines Lösungsmittels bei Temperaturen von weniger als 60°C zu 2,3,5,6-Tetrachlor-4-trifluormethyl-benzoylchlorid chloriert.
b) 2,3,5,6-Tetrachlor-4-trifluormethyl-benzoylchlorid mit Kaliumfluorid in Gegenwart eines aprotischen, dipolaren, inerten Lösungsmittels zu 2,3,5,6-Tetrafluro-4-trifluormethyl-benzoylfluorid fluoriert,
c) 2,3,5,6-Tetrafluor-4-trifluormethyl-benzoylfluorid zu 2,3,5,6-Tetrafluor-4-trifluormethyl-benzoesäure hydrolysiert,
d) 2,3,5,6-Tetrafluor-4-trifluormethyl-benzoesäure bei erhöhter Temperatur zu 2,3,5,6-Tetrafluorbenzotrifluorid decarboxyliert und
e) 2,3,5,6-Tetrafluor-benzotrifluorid mit Oleum zu 2,3,5,6-Tetraflurobenzoesäure verseift.
2) Verfahren nach Anspruch 1, dadurch gekennzeichnet, daß man die Produkte der Stufen b) und c) nicht isoliert.
3) Verfahren nach Ansprüchen 1 und 2, dadurch gekennzeichnet, daß man die Stufen b), c) und d) im gleichen Reaktionsgefäß unmittelbar nacheinander durchführt.
4) Verfahren nach Ansprüchen 1 bis 3, dadurch gekennzeichnet, daß man in Stufe a) Chlorsulfonsäure in einer Menge von 2 - 10 Mol pro Mol 4-Trifluormethyl-benzoylfluorid einsetzt und die Chlorierung in Gegenwart eines Lösungsmittels durchführt.
5) Verfahren nach Ansprüchen 1 bis 4, dadurch gekennzeichnet, daß man in Stufe b) 5 - 20 Mol Kaliumfluorid pro Mol 2,3,5,6-Tetrachlor-4-trifluormethyl-benzoylchlorid einsetzt.
6) Verfahren nach Ansprüchen 1 bis 5, dadurch gekennzeichnet, daß man in Stufe c) von 1 bis 1,5 Mol Wasser, bezogen auf Edukt einsetzt.
7) Verfahren nach Ansprüchen 1 bis 6, dadurch gekennzeichnet, daß man die Stufe d) bei Temperaturen von 100 bis 160°C zu Ende führt.
8) Verfahren nach Ansprüchen 1 bis 7, dadurch gekennzeichnet, daß man in Stufe e) von 10 bis 50 Gew.-% $SO_3$ enthaltendes Oleum in einer Menge von 1 Mol oder mehr, bezogen auf das Produkt der Stufe d) einsetzt und bei einer Temperatur von 70 bis 120°C arbeitet.
9) 2,3,5,6-Tetrachlor-4-trifluormethyl-benzoylchlorid.
10) 2,3,5,6-Tetrachlor-4-trifluormethyl-benzoylfluorid.

**Claims**

1. Process for the preparation of 2,3,5,6-tetraflurobenzoic acid, characterized in that
a) 4-trifluoromethyl-benzoyl fluoride is chlorinated in the presence of chlorosulphonic acid and if appropriate in the presence of a solvent at temperatures of lower than 60°C to give 2,3,5,6-tetrachloro-4-trifluoromethyl-benzoyl chloride,

b) 2,3,5,6-tetrachloro-4-trifluoromethyl-benzoyl chloride is fluorinated with potassium fluoride in the presence of an aprotic, dipolar, inert solvent to give 2,3,5,6-tetrafluoro-4-trifluoromethylbenzoyl fluoride,

c) 2,3,5,6-tetrafluoro-4-trifluoromethyl-benzoyl fluoride is hydrolyzed to give 2,3,5,6-tetrafluoro-4-trifluoromethyl-benzoic acid,

d) 2,3,5,6-tetrafluoro-4-trifluoromethyl-benzoic acid is decarboxylated at elevated temperature to give 2,3,5,6-tetrafluorobenzotrifluoride and

e) 2,3,5,6-tetrafluoro-benzotrifluoride is hydrolyzed with oleum to give 2,3,5,6-tetraflurobenzoic acid.

2. Process according to Claim 1, characterized in that the products of steps b) and c) are not isolated.

3. Process according to Claims 1 and 2, characterized in that the steps b), c) and d) are carried out in the same reaction vessel immediately after one another.

4. Process according to Claims 1 to 3, characterized in that, in step a), chlorosulphonic acid is employed in an amount of 2–10 mol per mol of 4-trifluoromethylbenzoyl fluoride and the chlorination is carried out in the presence of a solvent.

5. Process according to Claims 1 to 4, characterized in that, in step b), 5–20 mol of potassium fluoride are employed per mol of 2,3,5,6-tetrachloro-4-trifluoromethylbenzoyl chloride.

6. Process according to Claims 1 to 5, characterized in that, in step c), from 1 to 1.5 mol of water, relative to the educt, are employed.

7. Process according to Claims 1 to 6, characterized in that step d) is continued to completion at temperatures from 100 to 160°C.

8. Process according to Claims 1 to 7, characterized in that, in step e), oleum, containing from 10 to 50% by weight of $SO_3$, is employed in an amount of 1 mol or more, relative to the product of step d), and the reaction is carried out at a temperature of 70 to 120°C.

9. 2,3,5,6-Tetrachloro-4-trifluoromethyl-benzoyl chloride.

10. 2,3,5,6-Tetrafluoro-4-trifluoromethyl-benzoyl fluoride.

## Revendications

Procédé pour la fabrication d'acide 2,3,5,6-tétrafluorobenzoïque, caractérisé en ce que

a) on chlore le fluorure de 4-trifluorométhylbenzoyle en présence d'acide chlorosulfonique et éventuellement en présence d'un solvant à des températures de moins de 60°C en chlorure de 2,3,5,6-tétrachloro-4-trifluorométhylbenzoyle,

b) on fluore le chlorure de 2,3,5,6-tétrachloro-4-trifluorométhylbenzoyle par le fluorure de potassium en présence d'un solvant inerte dipolaire aprotique en fluorure de 2,3,5,6-tétrafluoro-4-trifluorométhylbenzoyle,

c) on hydrolyse le fluorure de 2,3,5,6-tétrafluoro-4-trifluorométhylbenzoyle en acide 2,3,5,6-tétrafluoro-4-trifluorométhylbenzoïque,

d) on décarboxyle l'acide 2,3,5,6-tétrafluoro-4-trifluorométhylbenzoïque à temperature élevée en 2,3,5,6-tétrafluorotrifluorométhylbenzène et

e) on saponifie le 2,3,5,6-tétrafluorotrifluorométhylbenzène par l'oléum en acide 2,3,5,6-tétrafluorobenzoïque.

2. Procédé selon la revendication 1, caractérisé en ce que l'on n'isole pas les produits des stades b) et c).

3. Procédé selon les revendications 1 et 2, caractérisé en ce que l'on effectue directement les stades b), c) et d) l'un après l'autres dans le même récipient de réaction.

4. Procédé selon les revendications 1 à 3, caractérisé en ce que l'on utilise l'acide chlorosulfonique dans le stade a) en quantité de 2–10 moles par mole de fluorure de 4-trifluorométhylbenzoyle et on effectue la chloration en présence d'un solvant.

5. Procédé selon les revendications 1 à 4, caractérisé en ce que l'on utilise dans le stade b) 5–20 moles de fluorure de potassium par mole de chlorure de 2,3,5,6-tétrachloro-4-trifluorométhylbenzoyle.

6. Procédé selon les revendications 1 à 5, caractérisé en ce que l'on utilise dans le stade c) de 1 à 1,5 mole d'eau par rapport au produit de départ.

7. Procédé selon les revendications 1 à 6, caractérisé en ce que l'on conduit à son terme le stade d) à des températures de 100 à 160°C.

8. Procédé selon les revendications 1 à 7, caractérisé en ce que l'on utilise dans le stade e) de l'oléum contenant 10 à 50% en poids de $SO_3$ en quantité d'une mole ou plus par rapport au produit du stade d) et on opère à une température de 70 à 120°C.

9. Le chlorure de 2,3,5,6-tétrachloro-4-trifluorométhylbenzoyle.

10. Le fluorure de 2,3,5,6-tétrafluoro-4-trifluorométhylbenzoyle.